**Europäisches Patentamt**

(19) **European Patent Office**

**Office européen des brevets**

(11) Veröffentlichungsnummer: **0 073 057**
**B1**

(12) # EUROPÄISCHE PATENTSCHRIFT

(45) Veröffentlichungstag der Patentschrift:
**09.12.87**

(51) Int. Cl.⁴: **A 61 F 2/30**

(21) Anmeldenummer: **82107734.4**

(22) Anmeldetag: **24.08.82**

(54) **Endoprothese.**

(30) Priorität: **28.08.81 DE 3134078**

(43) Veröffentlichungstag der Anmeldung:
**02.03.83 Patentblatt 83/9**

(45) Bekanntmachung des Hinweises auf die Patenterteilung:
**09.12.87 Patentblatt 87/50**

(84) Benannte Vertragsstaaten:
**CH FR GB LI NL SE**

(56) Entgegenhaltungen:
**FR - A - 2 248 820**
**US - A - 4 054 955**

**Die Akte enthält technische Angaben, die nach dem Eingang der Anmeldung eingereicht wurden und die nicht in dieser Patentschrift enthalten sind.**

(73) Patentinhaber: **GMT Gesellschaft für medizinische Technik mbH, Holstenstrasse 2, D-2000 Hamburg 50 (DE)**
Patentinhaber: **Waldemar Link GmbH & Co, Barkhausenweg 10, D-2000 Hamburg 63 (DE)**

(72) Erfinder: **Engelbrecht, Eckart, Andreasstrasse 33, D-2000 Hamburg 60 (DE)**
Erfinder: **Nieder, Elmar, Hinterdeich 117, D-2155 Jork (DE)**
Erfinder: **Keller, Arnold, An der Naherfurth 5, D-2061 Kaihude (DE)**

(74) Vertreter: **Heldt, Gert, Dr. Dipl.-Ing., Neuer Wall 59 III, D-2000 Hamburg 36 (DE)**

ACTORUM AG

EP 0 073 057 B1

## Beschreibung

Die Erfindung betrifft eine Endoprothese zum Ersatz eines in einem unbrauchbaren Lager eines Knochens nicht mehr schwenkbaren Knochenteiles mit mindestens einem an einem Ende eines Stiels befestigten Lagerkopf.

Endoprothesen der vorgenannten Art sind in verschiedenen Ausführungsarten bekannt. Umfangreiche Knochenzerstörungen verhindern jedoch in manchen Fällen den Einbau von Lagern, in denen beispielsweise ein beweglicher Knochen gegenüber seinem benachbarten Knochen drehbar und/oder schwenkbar gelagert ist. Beispielsweise können beckenseitige Knochenzerstörungen des Hüftgelenkes bei Korrekturoperationen eine Endoprotheseversorgung verhindern, wenn der Einbau standardisierter oder spezieller Hüftpfannen sowie Pfannenrekonstruktionen mit Hilfe von Verbundosteosynthesen oder Spongiosaplastiken unmöglich ist. Diese Situation führen zu teilweise unbefriedigenden Rückzugsoperationen, wie zum Beispiel Resektionsarthroplastiken in allen Variationen.

In diesen Fällen wurde bisher die sogenannte Girdelstone-Plastik angewendet, bei der der Oberschenkelknochen mit Hilfe von Bändern gegen den Beckenknochen gepresst wird. Bei dieser Art der Befestigung besteht lediglich ein Kraftschluss zwischen dem Oberschenkelknochen und dem Beckenknochen. In Richtung der vom Beckenknochen in den Oberschenkelknochen zu übertragender Kräfte besteht hingegen kein Formschluss. Aus diesem Grunde muss damit gerechnet werden, dass die verschiedenen Teile der Plastik sich gegeneinander verschieben und zu instabilen und schmerzhaften Zuständen führen.

Darüber hinaus sind Endoprothesen bekannt geworden, die im Falle von Lagerschädigungen dazu beitragen sollen, Luxationen zu verhindern. Dabei werden (FR-A-2 248 820) bewegungsbegrenzende Mittel eingesetzt, die verhindern sollen, dass die beiden über ein Schwenklager miteinander verbundenen Knochen sich ausserhalb eines Bereichs bewegen können, der innerhalb eines Dreh- beziehungsweise Schwenkkreises des gegenüber dem festen Knochen beweglichen Knochens liegt. Diese bewegungsbegrenzenden Mittel können beispielsweise als zwei ineinander greifende Ösen ausgebildet sein, von denen die eine mit einem Stiel versehen ist, der in den beweglichen Knochen hineinragt, während die andere mit einer Platte verbunden ist, die an dem Knochen befestigt wird, der mit dem beweglichen Knochen ein Dreh- beziehungsweise Schwenkgelenk ausbildet. Voraussetzung für die Funktion dieses Dreh- beziehungsweise Schwenklagers ist mithin, dass der Dreh- beziehungsweise Schwenkbegrenzer aus zwei Teilen besteht, von denen jedes an einem Knochen befestigt werden kann. Diese Endoprothese kann daher nicht in solchen Fällen eingesetzt werden, in denen mindestens einer der beiden Knochen soweit geschädigt ist, dass an ihm das Lagerteil einer Endoprothese nicht mehr befestigt werden kann.

Darüber hinaus ist eine weitere Endoprothese bekannt geworden (US-A-4 054 955), bei der die Luxation eines Gelenkes dadurch vermieden wird, dass der bewegliche Knochen in einer Führungskurve geführt wird, die zu diesem Zwecke zwischen dem beweglichen Knochen und dem ihm zugeordneten festen Knochen angeordnet wird. Diese Führungskurve lässt nur bestimmte Bewegungen des beweglichen Knochens zu. Die genaue Lage des beweglichen Knochens bezüglich dieser Führungskurve kann durch eine Vielzahl von Einstellmöglichkeiten bestimmt werden, die an der Endoprothese vorgesehen sind.

Bei diesen beiden Endoprothesen steht sowohl beim beweglichen Knochen als auch bei dem festen Knochen, gegenüber dem der bewegliche Knochen seine Bewegungen durchführt, genügend gesundes Knochenmaterial zur Verfügung, um die jeweils notwendigen Endoprothesenteile mit dem Knochen zu verbinden. Diese Prothesen eröffnen daher nicht die Möglichkeit, den beweglichen Knochen gegenüber dem festen Knochen auch dann noch beweglich zu lagern, wenn am festen Knochen nicht genügend gesunde Knochenmasse zur Verfügung steht, um in ihr ein Gelenkteil der Endoprothese zu verankern.

Aufgabe der vorliegenden Erfindung ist es daher, die Endoprothese der eingangs genannten Art so zu verbessern, dass der bewegliche Knochen im festen Knochen auch dann noch funktionsgerecht gelagert werden kann, wenn wegen einer fortgeschrittenen Schädigung des festen Knochens ein Gelenkteil an ihm nicht mehr befestigt werden kann.

Diese Aufgabe wird erfindungsgemäss dadurch gelöst, dass der Lagerkopf als Sattel ausgebildet ist, der in einer Mulde zwischen zwei sie begrenzenden Sattelhörnern liegt.

Durch diese Ausgestaltung des Lagerkopfes ist es möglich, den beweglichen Knochen an einem eigens zu seiner Lagerung vorgesehenen Drucklager zu lagern, das am festen Knochen an einer Stelle vorgesehen wird, die für die Übertragung der Druckkräfte ausreichend fest ist. Der Sattel ermöglicht einen Formschluss mit dem festen Knochen, in dem ein Loch vorgesehen wird, durch das der Sattel mit seinem längeren Sattelhorn hindurchgeführt wird. Dieser Formschluss verhindert, dass die beiden Teile sich unter dem Einfluss der Last gegeneinander verschieben. Der Träger der Endoprothese kann damit rechnen, dass die sich nach dem Einbau der Prothese für ihn ergebenden Verhältnisse nahezu konstant bleiben. Mit einer Abnutzung braucht er sowohl im Bereich des Knochens als auch in dem des Lagerkopfes kaum zu rechnen. Die beiden Teile sind in ihrer Gestaltung so aufeinander abgestimmt, dass sie weitgehend ohne Abrieb aufeinander arbeiten können. Darüber hinaus bildet sich am Knochen in vielen Fällen unter dem Einfluss der Belastung eine Knochenverdichtung aus, die den Knochen stabilisiert.

Gemäss einer bevorzugten Ausführungsform der Erfindung weist der Sattel im Bereich der Mulde eine vom Stiel weggerichtete Auswölbung auf.

Weitere Einzelheiten der Erfindung ergeben sich aus der nachfolgend ausführlichen Beschreibung und den beigefügten Zeichnungen, in denen eine bevorzugte Ausführungsform der Erfindung beispielsweise veranschaulicht.

Es zeigen:

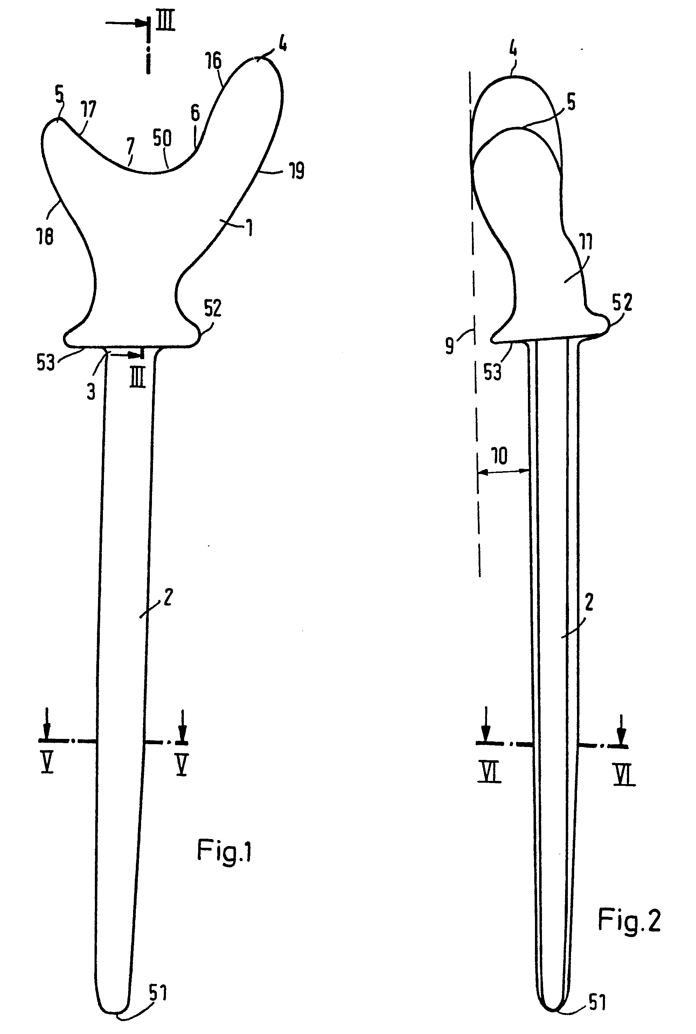

Fig. 1: Eine Draufsicht auf eine Endoprothese,

Fig. 2: eine Seitenansicht einer Endoprothese,

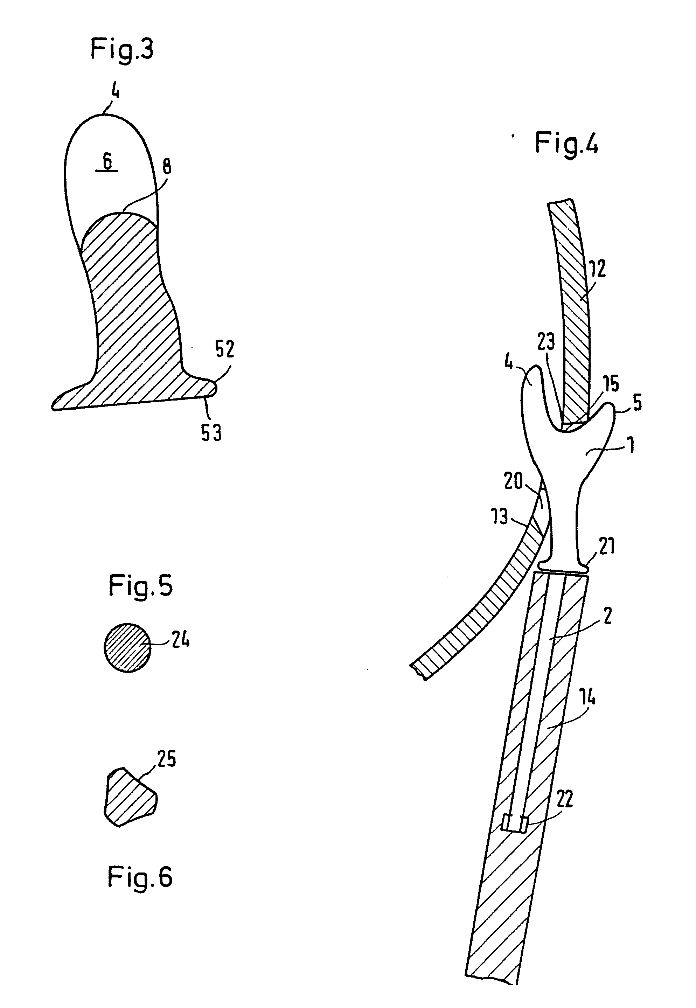

Fig. 3: einen Schnitt durch einen Lagerkopf entlang der Schnittlinie III–III in Fig. 1,

Fig. 4: einen Schnitt durch ein Einbaubeispiel für eine Endoprothese,

Fig. 5: einen Schnitt durch einen Stiel entlang der Schnittlinie V–V in Fig. 3 und

Fig. 6: einen Schnitt durch einen Stiel anderen Querschnitts entlang der Schnittlinie VI–VI in Fig. 2.

Eine Endoprothese besteht im wesentlichen aus einem Lagerkopf 1 und einem Stiel 2. Der Lagerkopf 1 ist mit dem Stiel 2 fest verbunden. Am Übergang vom Stiel 2 zum Lagerkopf 1 ist ein Kragen 3 vorgesehen. Der Kragen ist als ein vom Stiel 2 wegweisender Wulst 52 ausgebildet. Dieser Wulst 52 ist auf seiner dem Stiel 2 zugewandten Unterseite 53 als eine Auflagefläche ausgebildet.

Auf seiner dem Stiel 2 gegenüber liegender Seite ist der Lagerkopf 1 mit zwei Sattelhörnern 4, 5 versehen, von denen das eine länger und das andere kürzer in eine vom Stiel 2 weggerichtete Richtung weisen. Die beiden Sattelhörner 4, 5 schliessen zwischen sich einen Sattel 6 ein, der vom höheren Sattelhorn 4 durch eine Mulde 7 bis zum kürzeren Sattelhorn 5 verläuft. Im Bereich der Mulde 7 weist der Sattel 6 eine kreisförmige Auswölbung 8 in Richtung auf die Sattelhörner 4, 5 auf. Die kreisförmige Begrenzungslinie dieser Auswölbungslinie 8 flacht sich in Richtung auf die Sattelhörner 4, 5 stetig ab, so dass sie im Bereich der Sattelhörner 4, 5 nur noch flach in Richtung auf einander zu gewölbt ist.

Die Sattelhörner 4, 5 spannen mit ihren seitlichen Begrenzungsflächen eine gedachte Ebene 9 auf, die in etwa planparallel zur Mittelachse des Stiels 2 verläuft. Zwischen der gedachten Ebene 9 und einer ihr zugewandten Aussenfläche des Stiels 2 liegt ein Abstand 10, dessen Länge einer Abkröpfung 11 entspricht, die im Bereich des Übergangs der Sattelhörner 4, 5 in den Stiel 2 vorgesehen ist. Diese Abkröpfung 11 des Lagerkopfes 1 trägt der Tatsache Rechnung, dass die Endoprothese insbesondere im Bereich eines Beckenknochens 12 so weit ausserhalb einer Auswölbung 13 dieses Beckenknochens 12 verlaufen muss, dass Schwenkbewegungen eines mit dem Stiel 2 verbundenem Knochenteils 14 möglich sind, ohne dass dieses Knochenteil 14 beziehungsweise die mit ihm verbundene Endoprothese im Bereich der Auswölbung 13 den Beckenknochen 12 berührt. Andererseits muss der Stiel 2 bei einem in den Beckenknochen 12 eingesetzten Lagerkopf 1 in Richtung des natürlichen Verlaufs des Knochenteils 14 ausgerichtet sein.

Die Mulde 7 erstreckt sich in Form einer kreisförmigen Begrenzungsfläche 15 von einem Sattelhorn 4 zum anderen Sattelhorn 5. In diese kreisförmige Begrenzungsfläche 15 münden die Sattelhörner 4, 5 tangential miteinander zugewandten Innenfläche 16, 17 ein. Der Verlauf der Sattelhörner 4, 5 in bezug auf den Stiel 2 ist so festgelegt, dass die Mittellinien, die jeweils durch die Sattelhörner 4, 5 gelegt werden, einen Winkel von etwa 30° mit einer gedachten Verlängerung des Stiels 2 bilden.

Die Sattelhörner 4, 5 münden mit ihren den Innenflächen 16, 17 abgewandten Aussenflächen 18, 19 in die Abkröpfung 11 ein. Der Verlauf der Aussenflächen 18, 19 ist so festgelegt, dass sie etwa dem Verlauf von Kraftlinien entsprechen, die sich unter der Einwirkung des auf den Lagerkopf 1 wirkenden Druckes in diesem ausbilden. Die Sattelhörner 4, 5 sind an ihren der Mulde 7 abgewandten Enden in einer quer zur Ebene 9 verlaufenden Richtung kreisförmig abgerundet. Zum Einbau der Endoprothese wird ein Loch 20 in den Knochen, beispielsweise in den Beckenknochen 12 eingebracht; häufig ist es allerdings schon vorhanden durch vorangegangene Zerstörungen. Durch dieses Loch 20 wird eines der beiden Sattelhörner 4, 5 so hindurchgesteckt, dass der Stiel 2 auf der Seite des Beckenknochens 12 verläuft, die dem Knochenteil 14 benachbart ist, in das der Stiel 2 eingebracht werden muss. Das Einsetzen des Sattelhorns 4 geschieht aber erst, nachdem der Stiel 2 im Knochenteil 14 fest verankert worden ist. Diese Verankerung geschieht zweckmässigerweise mit Knochenzement. Der Stiel 2 ragt so weit in den Knochenteil 14 hinein, dass dieser mit seinem dem Beckenknochen 12 zugewandten oberen Abschluss 21 am Kragen 3 der Abkröpfung 11 aufliegt. Falls der Kragen 3 von einem Wulst 52 umgeben ist, beaufschlagt der obere Abschluss 21 die Unterseite 53. Auf diese Weise ist dafür gesorgt, dass die Druckspannungen vom Lagerkopf 1 über den Kragen 3 und dem Stiel 2 ohne Schwierigkeiten in den Knochenteil 14 eingeleitet werden können. Für die richtige Justierung des Stiels 2 im Knochenteil 14 sorgt eine sternförmige Zielvorrichtung 22, dessen einzelnen sternförmigen Verlängerungen sich beim Hineinschieben des Stiels 2 in den Knochenteil 14 den richtigen Weg bis zu ihrem endgültigen Stand bahnen.

Nach der Verankerung des Stiels 2 im Knochenteil 14 wird der Lagerkopf 1 so in das Loch 20 eingeführt, dass der im Stiel 2 angewandte obere Teil 23 des Loches 20 die Mulde 7 beaufschlagt. Die Beaufschlagung der Mulde 7 erfolgt im Bereich eines tiefsten Punktes 50 der kreisförmigen Begrenzungsfläche 15. Dieser tiefste Punkt 50 ist dem durch das Loch 20 hindurchragenden Sattelhorn 4 bzw. 5 benachbart. Im Regelfall wird das längere Sattelhorn 4 von aussen durch das Loch 20 hindurchgeführt, so dass der tiefste Punkt 50 dem längeren Sattelhorn 4 benachbart ist. Es ist aber auch denkbar, statt des längeren Sattelhorns 4 das kürzere Sattelhorn 5 durch das Loch 20 hindurchragen zu lassen. In diesem Falle ist der tiefste Punkt 50 dem kürzeren Sattelhorn 5 benachbart. In jedem Falle ist der tiefste Punkt 50 gegenüber einer gedachten Verlängerung der Mittelachse des Stiels 2 in Richtung auf das durch das Loch 20 hindurchragende Sattelhorn 4 bzw. 5 verschoben. Es ist aber auch denkbar, beide Sattelhörner 4, 5 gleichlang auszubilden.

Das Loch 20 wird an einer Stelle des Beckenknochens 12 vorgesehen, die möglichst gut erhalten und damit in der Lage ist, die auftretenden Druckspannungen zu übertragen. Auf diese Weise ist es erforderlich, dass die im Beckenknochen 12 zu befestigende Endoprothese sich der jeweiligen Lage des Loches 12 anpassen kann, um insoweit sich dem jeweiligen individuellen Bedarf anpassen zu können, kann die Abkröpfung 11 länger oder kürzer sein. Es ist auch möglich, den Lagerkopf 1 gegenüber dem Stiel 2 so zu verdrehen, dass die gedachte Ebene 9 in Richtung der Abkröpfung 11 verläuft. Schliesslich ist es denkbar, den Lagerkopf 1 gegenüber dem Stiel 2 so zu verdrehen, dass die gedachte Ebene 9 mit einer sich durch die Abkröpfung 11 erstreckenden anderen gedachten Ebene jedem beliebigen und dem jeweiligen Einsatzzweck angepassten Winkel bildet.

Der obere Teil 23 des Loches 20 ist als eine Kante ausgebildet. Diese Kante kann der erfahrene Chirurg so gestalten, dass sie mit einer möglichst grossen Fläche der Mulde 7 aufliegt.

Im Regelfall wird die Endoprothese so eingesetzt, dass diese Kante in etwa quer zu deren von den Sattelhörnern 4, 5 aufgespannten gedachten Ebenen 9 verläuft.

Der Stiel 2 kann einen runden Querschnitt 24 aufweisen. Um eine zusätzliche Sicherheit gegen Verdrehen des Stiels 2 innerhalb des Knochenteils 14 zu erzeugen, kann der Querschnitt des Stiels 2 auch jede andere Gestaltung aufweisen. Beispielsweise kann er an drei gleichmässig über den Querschnitt verteilten Stellen mit Abflachungen 25 versehen sein. Der Stiel 2 kann mit einer zylindrischen Oberfläche versehen sein. Es ist aber auch möglich, den Stiel 2 in Richtung auf eine dem Lagerkopf 1 abgewandte Spitze 51 konisch zulaufen zu lassen. In diesem Fall ist lediglich der obere, dem Lagerkopf 1 benachbarte Teil des Stiels 2 zylindrisch ausgebildet. Die konische Ausbildung des Stiels 2 erleichtert die Einführung des Stiels 2 in den Knochenteil 14.

Das Material für die Endoprothese besteht aus einer Legierung aus Chrom, Kobalt und Molybdän.

Die Endoprothese kann Teil eines Systems sein, das der endoprothetischen Versorgung des Oberschenkels dient. So kann sie kombiniert werden mit einem partiellen proximalen Femurersatz oder mit einem totalen Femurersatz.

**Patentansprüche**

1. Endoprothese zum Ersatz eines in einem unbrauchbaren Lager eines Knochens (12) nicht mehr schwenkbaren Knochenteiles (14) mit mindestens einem an einem Ende eines Stiels (2) befestigten Lagerkopf (1), dadurch gekennzeichnet, dass der Lagerkopf (1) als Sattel (6) ausgebildet ist, der in einer Mulde (7) zwischen zwei sie begrenzenden Sattelhörnern (4, 5) liegt.

2. Endoprothese nach Anspruch 1, dadurch gekennzeichnet, dass der Sattel (6) im Bereich der Mulde (7) eine vom Stiel (2) weggerichtete Auswölbung (8) aufweist.

3. Endoprothese nach Anspruch 1 und 2, dadurch gekennzeichnet, dass die Mulde (7) einen quer zu einer von den Sattelhörnern (4, 5) und dem Sattel (6) aufgespannten Ebene (9) liegenden Querschnitt etwa in Form eines Kreissektors aufweist.

4. Endoprothese nach Anspruch 1 bis 3, dadurch gekennzeichnet, dass die Mulde (7) auf ihrem zwischen den Sattelhörnern (4, 5) liegenden Grund in Form einer kreisförmigen Begrenzungsfläche (15) ausgebildet ist, in die die Sattelhörner (4, 5) mit ihren einander zugewandten Innenflächen (16, 17) tangential einmünden.

5. Endoprothese nach Anspruch 1 bis 4, dadurch gekennzeichnet, dass die Sattelhörner (4, 5) an ihren der Mulde (7) abgewandten Enden in einer quer zur Ebene (9) verlaufenden Richtung kreisförmig abgerundet sind.

6. Endoprothese nach Anspruch 1 bis 5, dadurch gekennzeichnet, dass die Sattelhörner (4, 5) mit ihren den Innenflächen (16, 17) abgewandten Aussenflächen (18, 19) den Lagerkopf (1) begrenzen.

7. Endoprothese nach Anspruch 1 bis 6, dadurch gekennzeichnet, dass der Lagerkopf (1) im Bereich seiner Einmündung in den Stiel (2) als Kragen (3) ausgebildet ist, der auf seiner dem Lagerkopf (1) abgewandten Seite als Auflagerfläche ausgebildet ist, in die der um die Breite des Kragens (3) schlankere Stiel (2) eingesetzt ist.

8. Endoprothese nach Anspruch 1 bis 7, dadurch gekennzeichnet, dass der Kragen (3) je nach Länge des zu ersetzenden Knochenteils (14) weiter oder weniger weiter entfernt vom Sattel (6) am Stiel (2) angeordnet ist.

9. Endoprothese nach Anspruch 1 bis 8, dadurch gekennzeichnet, dass der Kragen (3) als ein vom Stiel (2) wegweisender Wulst (52) ausgebildet ist.

10. Endoprothese nach Anspruch 1 bis 9, dadurch gekennzeichnet, dass die Sattelhörner (4, 5) von ungleicher Länge sind.

11. Endoprothese nach Anspruch 1 bis 10, dadurch gekennzeichnet, dass die Aussenflächen (18, 19) in den Kragen (3) einmünden.

12. Endoprothese nach Anspruch 1 bis 11, dadurch gekennzeichnet, dass der Stiel (2) einen runden Querschnitt aufweist.

13. Endoprothese nach Anspruch 1 bis 11, dadurch gekennzeichnet, dass der Stiel (2) einen mehrkantigen Querschnitt aufweist.

14. Endoprothese nach Anspruch 1 bis 13, dadurch gekennzeichnet, dass der Stiel (2) an seinem vom Lagerkopf (1) abgewandten Ende eine das Einsetzen erleichternde Zielvorrichtung (22) aufweist.

15. Endoprothese nach Anspruch 12, dadurch gekennzeichnet, dass der Stiel (2) eine zylindrische Aussenfläche aufweist.

16. Endoprothese nach Anspruch 12, dadurch gekennzeichnet, dass der Stiel (2) in seinem dem Lagerkopf (1) benachbarten oberen Bereich eine zylindrische Aussenfläche aufweist, die in Richtung auf eine dem Lagerkopf (1) abgewandte Spitze (51) konisch zuläuft.

17. Endoprothese nach Anspruch 1 bis 16, dadurch gekennzeichnet, dass der Lagerkopf (1) im Bereich eines zwischen dem Kragen (3) und der Mulde (7) liegenden Teiles eine Abkröpfung (11) aufweist.

18. Endoprothese nach Anspruch 1 bis 17, dadurch gekennzeichnet, dass die Abkröpfung (11) eine einem individuellen Einbauzweck anpassbare Länge aufweist.

19. Endoprothese nach Anspruch 1 bis 18, dadurch gekennzeichnet, dass die die Mulde (7) zwischen den Sattelhörnern (4, 5) begrenzende kreisförmige Begrenzungsfläche (15) einen dem individuellen Einbauzweck anpassbaren Durchmesser aufweist.

20. Endoprothese nach Anspruch 1 bis 19, dadurch gekennzeichnet, dass die kreisförmige Begrenzungsfläche (15) im Bereich des längeren Sattelhorns (4) ihren tiefsten Punkt (50) aufweist.

21. Endoprothese nach Anspruch 1 bis 20, dadurch gekennzeichnet, dass der tiefste Punkt (50) gegenüber einer gedachten Verlängerung der Mittelachse des Stiels (2) in Richtung auf das längere Sattelhorn (4) verschoben ist.

22. Endoprothese nach Anspruch 1 bis 21, dadurch gekennzeichnet, dass die von den Sattelhörnern (4, 5) aufgespannte gedachte Ebene (9) um die Länge der Abkröpfung (11) versetzt parallel zur Längsachse des Stiels (2) verläuft.

23. Endoprothese nach Anspruch 1 bis 22, dadurch gekennzeichnet, dass die Abkröpfung (11) in der von den Sattelhörnern (4, 5) aufgespannten gedachten Ebene verläuft.

24. Endoprothese nach Anspruch 1 bis 23, dadurch gekennzeichnet, dass die von den Sattelhörnern (4, 5) aufgespannte gedachte Ebene (9) mit einer von der Abkröpfung (11) und dem Stiel (2) aufgespannten Ebene einen den individuellen Einbauzwecken angepassten Winkel bildet.

## Claims

1. Endoprosthesis for replacing a bone part (14) no longer pivotable in an unusable bearing of a bone (12), having at least one bearing head (1) fixed to one end of a shank (2), characterized in that the bearing head (1) is constructed as a saddle (6), which is located in a depression (7) between two saddle projections or horns (4, 5) bounding it.

2. Endoprosthesis according to claim 1, characterized in that the saddle (6) has a curvature (8) directed away from shank (2) in the vicinity of depression (7).

3. Endoprosthesis according to claims 1 and 2, characterized in that the depression (7) has a cross-section roughly shaped like a circular sector at right angles to a plane (9) covered by the saddle horns (4, 5) and saddle (6).

4. Endoprosthesis according to claims 1 to 3, characterized in that on its base located between the saddle horns (4, 5), depression (7) is constructed in the form of a circular bounding face (15), into which tangentially issue the saddle horns (4, 5) with their facing inner surfaces (16, 17).

5. Endoprosthesis according to claims 1 to 4, characterized in that at their ends remote from depression (7), the saddle horns (4, 5) are circularly rounded in a direction at right angles to plane (9).

6. Endoprosthesis according to claims 1 to 5, characterized in that the saddle horns (4, 5) bound with their outer surfaces (18) remote from their inner surfaces (16, 17) the bearing head (1).

7. Endoprosthesis according to claims 1 to 6, characterized in that in the vicinity of its entry into shank (2), bearing head (1) is constructed as a collar (3), which is constructed as a bearing surface on its side remote from the bearing head (1) and into which is inserted the shank (2) which is made more slender by the width of collar (3).

8. Endoprosthesis according to claims 1 to 7, characterized in that, as a function of the length of the bone part (14) to be replaced, collar (3) is positioned on shank (2) at a greater or smaller distance from saddle (6).

9. Endoprosthesis according to claims 1 to 8, characterized in that the collar (3) is constructed as a bead (52) directed away from shank (2).

10. Endoprosthesis according to claims 1 to 9, characterized in that the saddle horns (4, 5) are of unequal length.

11. Endoprosthesis according to claims 1 to 10, characterized in that the outer surfaces (18, 19) issue into collar (3).

12. Endoprosthesis according to claims 1 to 11, characterized in that the shank (2) has a circular cross-section.

13. Endoprosthesis according to claims 1 to 11, characterized in that shank (2) has a many-sided cross-section.

14. Endoprosthesis according to claims 1 to 13, characterized in that at its end remote from bearing head (1), shank (2) has sight (22) facilitating insertion.

15. Endoprosthesis according to claim 12, characterized in that shank (2) has a cylindrical outer surface.

16. Endoprosthesis according to claim 12, characterized in that in its upper region adjacent to bearing head (1), shank (2) has a cylindrical outer surface, which tapers towards a tip (51) remote from bearing head (1).

17. Endoprosthesis according to claims 1 to 16, characterized in that in the vicinity of a part located between collar (3) and depression (7), bearing head (1) has a bend (11).

18. Endoprosthesis according to claims 1 to 17, characterized in that the bend (11) has a length adaptable to an individual intended use.

19. Endoprosthesis according to claims 1 to 18, characterized in that the depression (7) has a diameter adaptable to the individual intended use

5

between the circular bounding surface (14) bounding the saddle horns (4, 5).

20. Endoprosthesis according to claims 1 to 19, characterized in that the circular bounding surface (15) has its lowest point (50) in the vicinity of the longer saddle horn (4).

21. Endoprosthesis according to claims 1 to 20, characterized in that the lowest point (50) is displaced towards the longer saddle horn (4) with respect to an imaginary extension of the central axis of shank (2).

22. Endoprosthesis according to claims 1 to 21, characterized in that the imaginary plane (9) covered by the saddle horns (4, 5) is displaced parallel to the longitudinal axis of shank (2) by the length of bend (11).

23. Endoprosthesis according to claims 1 to 22, characterized in that bend (11) runs in the imaginary plane covered by saddle horns (4, 5).

24. Endoprosthesis according to claims 1 to 23, characterized in that the imaginary plane (9) covered by saddle horns (4, 5) forms, together with a plane covered by bend (11) and shank (2), an angle adapted to the individual intended uses.

**Revendications**

1. Endoprothèse pour le remplacement d'une partie d'os (14) ne pouvant plus pivoter dans un palier inutilisable d'un os (12), munie d'au moins une tête de palier (1) fixée à une extrémité d'un manche (2), caractérisée par le fait que la tête de palier (1) est conçue sous forme de selle (6) qui est située dans un creux (7) entre deux cornes de selle (4, 5) qui la limitent.

2. Endoprothèse selon la revendication 1, caractérisée par le fait que la selle (6) présente dans la région du creux (7) une convexité (8) dirigée à l'opposé du manche (2).

3. Endoprothèse selon les revendications 1 et 2, caractérisée par le fait que le creux (7) présente une section à peu près en forme de secteur circulaire située transversalement à un plan (9) tendu par les cornes de selle (4, 5) et la selle (6).

4. Endoprothèse selon l'une des revendications 1 à 3, caractérisée par le fait que le creux (7), sur son fond situé entre les cornes de selle (4, 5), est conçu en forme de surface circulaire de limitation (15) dans laquelle les cornes de selle (4, 5) débouchent tangentiellement par leurs surfaces intérieures tournées l'une vers l'autre.

5. Endoprothèse selon l'une des revendications 1 à 4, caractérisée par le fait que les cornes de selle (4, 5), à leurs extrémités opposées au creux (7), sont arrondies circulairement dans une direction située transversalement au plan (9).

6. Endoprothèse selon l'une des revendications 1 à 5, caractérisée par le fait que les cornes de selle (4, 5), par leurs surfaces extérieures (18, 19) opposées aux surfaces intérieures, (16, 17), limitent la tête de palier (1).

7. Endoprothèse selon l'une des revendications 1 à 6, caractérisée par le fait que la tête de palier (1), dans la région où elle se raccorde au manche (2) est conçue sous forme de collet (3) qui, sur son côté opposé à la tête de palier (1), est conçue sous forme de surface d'appui dans laquelle est inséré le manche (2), plus mince à raison de la largeur du collet (3).

8. Endoprothèse selon l'une des revendications 1 à 7, caractérisée par le fait que le collet (3), selon la longueur de la partie d'os (14) à remplacer, est disposé, sur le manche (2), plus ou moins loin de la selle (6).

9. Endoprothèse selon l'une des revendications 1 à 8, caractérisée par le fait que le collet (3) est constitué comme un bourrelet (52) se dirigeant à l'opposé du manche (2).

10. Endoprothèse selon l'une des revendications 1 à 9, caractérisée par le fait que les cornes de selle (4, 5) sont de longueur inégale.

11. Endoprothèse selon l'une des revendications 1 à 10, caractérisée par le fait que les surfaces extérieures (18, 19) débouchent dans le collet (3).

12. Endoprothèse selon l'une des revendications 1 à 11, caractérisée par le fait que le manche (2) présente une section ronde.

13. Endoprothèse selon l'une des revendications 1 à 11, caractérisée par le fait que le manche (2) présente une section polygonale.

14. Endoprothèse selon l'une des revendications 1 à 13, caractérisée par le fait que le manche (2) présente, à son extrémité opposée à la tête de palier, un dispositif de visée (22) facilitant l'insertion.

15. Endoprothèse selon la revendication 12, caractérisée par le fait que le manche (2) présente une surface extérieure cylindrique.

16. Endoprothèse selon la revendication 12, caractérisée par le fait que le manche (2) présente, dans sa région supérieure voisine de la tête de palier, une surface extérieure cylindrique qui se termine coniquement en direction d'une pointe (51) opposée à la tête de palier (1).

17. Endoprothèse selon l'une des revendications 1 à 16, caractérisée par le fait que la tête de palier (1) présente un décrochement (11) dans la région d'une partie située entre le collet (3) et le creux (7).

18. Endoprothèse selon l'une des revendications 1 à 17, caractérisée par le fait que le décrochement (11) présente une longueur pouvant être adaptée au but individuel d'installation.

19. Endoprothèse selon l'une des revendications 1 à 18, caractérisée par le fait que la surface limite circulaire (15) limitant le creux (7) entre les cornes de selle (4, 5) présente un diamètre adaptable au but individuel d'installation.

20. Endoprothèse selon l'une des revendications 1 à 19, caractérisée par le fait que la surface circulaire de limitation (15) présente son point le plus bas (50) dans la région de la plus longue (4) des cornes de selle.

21. Endoprothèse selon l'une des revendications 1 à 20, caractérisée par le fait que le point le plus bas (50) est décalé en direction de la plus longue (4) des cornes de selle relativement à une prolongement imaginaire de l'axe médian du manche (2).

22. Endoprothèse selon l'une des revendications 1 à 21, caractérisée par le fait que le plan imaginaire (9) mené par les cornes de selle (4, 5) est dirigé parallèlement à l'axe longitudinal du manche (2) avec décalage à raison de la longueur du décrochement.

23. Endoprothèse selon l'une des revendications 1 à 22, caractérisée par le fait que le décrochement (11) est situé dans le plan imaginaire mené par les cornes de selle (4, 5).

24. Endoprothèse selon l'une des revendications 1 à 23, caractérisée par le fait que le plan imaginaire (9) mené par les cornes de selle (4, 5) fait, avec un plan mené par le décrochement (11) et le manche (2), un angle adapté aux buts individuels d'installation.

Fig.1

Fig.2

Fig.3

Fig.4

Fig.5

Fig.6